# EUROPEAN PATENT APPLICATION

(11) **EP 4 385 552 A1**
(43) Date of publication of application: **19.06.2024**
(21) Application number: 23217350.0
(22) Date of filing: 15.12.2023
(51) Int. Cl.: A61M 39/06, A61B 17/22, A61B 17/34, A61M 1/00, A61B 17/221

(54) **AN INTRODUCER ASSEMBLY HAVING A LOW-PROFILE ACCESS SHEATH AND A HUB ASSEMBLY**

(30) Priority: 16.12.2022 US 202263387863 P
(71) Applicant: Cook Medical Technologies LLC, Bloomington, IN 47404 (US)
(72) Inventor: JONES, Matthew, West Lafayette, 47906 (US); OBERMILLER, Joseph, West Lafayette, 47906 (US); DOWELL, Angela R., Lafayette, 47904 (US); PEDERSEN, Wesley, Bloomington, 47403 (US); MAY, John, Mason, 45050 (US); FERRARI, Eugene M, Crown Point, 46307 (US); PUCKETT, Daniel, Dallas, 75205 (US); ISCH, Andrew P., Lafayette, 47905 (US); NEFF, Gary, Bloomington, 47408 (US); SANTELIZ AGUAYO, Erlan A., Indianapolis, 46222 (US); MCGOFF, Adam, Indianapolis, 46240 (US)
(74) Representative: Leach, Sean Adam

(57) **Abstract**

The present invention relates to an introducer assembly. The introducer assembly includes a sheath (12) and a hub assembly (20) interconnected with the proximal end of the sheath, the hub assembly (20) including a body defining an interior passageway in fluid communication with the lumen of the sheath, the body having a proximal end and a distal end. A valve assembly (30) is positioned within the interior passageway of the body, the valve assembly having a valve member, the valve member being at least partially movable from a first position to a second position with respect to the valve collar. A vacuum chamber (42) positioned distal to the valve assembly and in fluid communication with the interior chamber of the body and the lumen of the sheath, the vacuum chamber including a vacuum port (40).

## Description

### RELATED APPLICATIONS

This application claims the benefit of U.S. Provisional Application No. 63/387,863, entitled "An Introducer Assembly Having A Low-Profile Access Sheath and A Hub Assembly" and filed December 16, 2022, which is hereby incorporated herein by reference in its entirety.

### BACKGROUND

### 1. Technical Field

The present disclosure relates generally to the field of medical devices, and more particularly, the disclosure relates to medical devices, methods and kits useful in the disruption and removal of unwanted materials, such as calculi and other formations, from within body lumens.

### 2. Background Information

There is a continuing need for instruments to diagnose and treat people by means of minimally-invasive surgical procedures. For example, various organs and passages in the body are subject to the development of stones, calculi and the like. Kidney stones are a common problem across the globe. Kidney stones are painful and are the most frequent cause of kidney inflammation. Similarly, stones, calculi, concretions and the like can develop throughout the renal or urinary system, not only in the ureters and distal to them, but also in the renal tubules and in the major and minor renal calyxes.

Minimally invasive surgical procedures have been developed for the removal of stones, calculi, concretions and the like from the biliary, vascular, and urinary systems, as well as for the removal or retrieval of foreign bodies from a variety of locations in the body. Such procedures avoid the performance of open surgical procedures such as, for example, an anatrophic nephrolithotomy.

Access sheaths, such as ureteral access sheaths, may be used to gain access to body cavities through lumens during endoscopic and laparoscopic surgery, and by other procedures that generally use minimally invasive techniques. Ureteral access sheaths may be used with an endoscope for finding and removing kidney stones, and may be used in other applications, such as tissue biopsy and removal or diagnostic visualization, for example. Other applications for which an access sheath has been used include vascular procedures, as well as procedures requiring gastro-intestinal access (including bile ducts), uterine access, and bronchial access, for example. At least some endoscopes, hysteroscopes, sigmoidoscopes, bronchoscopes, and other types of instruments for minimally-invasive techniques may utilize such access sheaths.

Using a sheath provides for a way to protect the tissues of a patient during a procedure. For instance, if a kidney stone is to be removed, a retrieval basket may require many passages back and forth across a patient's ureter to remove stone fragments. A wire basket or other device is first passed through the ureter to retrieve stone fragments, and then passes back through to remove the captured fragments. Passing the basket through an access sheath instead of the ureter itself avoids trauma to the ureter and surrounding tissues; furthermore, the sheath mitigates the delay of regaining access at the ureteropelvic junction for each pass.

It would be highly desirable to have a device suitable for use during such procedures to improve efficiency. It would further be highly desirable to provide a device to the user to provide access to suction along with increased control the flow of suction while performing such procedures.

### BRIEF SUMMARY

Various exemplary medical devices and methods are described and illustrated herein.

In one aspect, an introducer assembly includes a sheath comprising a proximal end, a distal end, and at least one lumen disposed therethrough. A hub assembly is interconnected with the proximal end of the sheath, the hub assembly including a body defining an interior passageway in fluid communication with the lumen of the sheath, the body having a proximal end and a distal end. A valve assembly is positioned within the interior chamber of the body, the valve assembly comprising a valve member, the valve member being at least partially movable from a first position to a second position. A vacuum chamber is positioned distal to the valve assembly and in fluid communication with the interior chamber of the body and the lumen of the sheath, the vacuum chamber including a vacuum port. In one embodiment, the sheath includes an inner layer having an inner diameter and an outer layer having an outer diameter, the outer diameter of the outer layer being greater than the inner diameter of the inner layer by at most, about 1.2 Fr. In another embodiment, the valve member comprises a first button comprising a proximal portion, a distal portion and a first strut positioned between the proximal portion and the distal portion and a second button comprising a proximal portion a distal portion and a first strut positioned between the proximal portion and the distal portion.

In another aspect, a hub assembly for an introducer assembly, includes a body defining an interior chamber in fluid communication with the lumen of the sheath, the body having a proximal end and a distal end. A valve assembly is positioned within the interior chamber of the body, the valve assembly comprising, a valve collar positioned within the interior chamber of the body and defining an interior passageway. A valve member is connected to the valve collar, the valve member being at least partially movable from a first position to a second position with respect to the valve collar. A vacuum chamber is interconnected with the distal end of the valve collar and in fluid communication with the interior chamber of the body, the vacuum chamber including a vacuum port. In one embodiment, the valve member comprises the valve member comprises a first button having a first surface and a second surface and a second button having a first surface and a second surface.

In yet another aspect, an introducer assembly includes a multi-layer sheath comprising a proximal end, a distal end, and at least one lumen disposed therethrough, the sheathhaving an inner layer having an outer diameter, an outer layer having an outer diameter, and at least one coil embedded between the inner layer and the outer layer. A hub assembly is interconnected with the proximal end of the multi-layer sheath and having a proximal end, a distal end, and a body defining an interior chamber in fluid communication with the lumen of the sheath, the body having a proximal end and a distal end. A valve assembly is positioned within the interior chamber of the body. The valve assembly includes a valve collar positioned within the interior chamber of the body and defining an interior passageway, the interior passageway being in fluid communication with the at least one lumen of the sheath. A valve member is connected to the valve collar, the valve member being at least partially movable from a first position to a second position with respect to the valve collar. A vacuum chamber is interconnected with the distal end of the valve collar and in fluid communication with the interior chamber of the body and the lumen of the sheath, the vacuum chamber including a vacuum port.

### BRIEF DESCRIPTION OF THE DRAWINGS

FIG. 1 illustrates an embodiment of a low-profile access sheath with hub assembly.
FIG. 2 illustrates a top view of an embodiment to FIG. 1 of a low-profile access sheath with hub assembly.
FIG. 3 illustrates a prospective view of an embodiment of a hub assembly of the access sheath of FIG. 1.
FIG. 4 is an exploded view of the embodiment of the hub assembly of FIG. 3.
FIG. 5 illustrates an alternative embodiment of the hub assembly of FIG. 3 further comprising an additional medical device.
FIG. 6 illustrates a cross-sectional view of an embodiment of the access sheath of FIG. 1.
FIGS. 7A and 7B illustrate a schematic demonstrating the flow of fluid through an embodiment of the hub assembly when the valve assembly is open and closed.
FIG. 8 illustrates an alternative embodiment of a hub assembly for a low access sheath.
FIG. 9 illustrates an alternative embodiment of a low-profile access sheath with hub assembly.
FIGS. 10A and 10B illustrate a cross-sectional view of alternative embodiments of a button for use with a valve assembly of a hub assembly.
FIGS. 11A-11C illustrate an alternative embodiment of a hub assembly.
FIGS. 12A and 12B illustrate an alternative embodiment of a low-profile access sheath with hub assembly.
FIGS. 13A and 13B illustrate an embodiment of a proximal section of a body of the hub assembly of FIGS. 12A and 12B.
FIG. 14 illustrates an alternative embodiment of a distal section of a body of the hub assembly of FIGS. 12A and 12B.
FIGS. 15A and 15B illustrate an alternative embodiment of a button for use with a valve assembly of the hub assembly of FIGS. 12A and 12B.
FIGS. 16A and 16B illustrate cross-sectional views of the hub assembly of FIGS. 12A and 12B.
FIGS. 17A-17C illustrate cross-sectional views of the hub assembly of FIGS. 12A and 12B and a medical device disposed therethrough.
FIGS. 18A and 18B illustrate an alternative embodiment of a button for use with a valve assembly of a hub assembly.
FIGS. 19A and 19B illustrate an alternative embodiment of a button for use with a valve assembly of a hub assembly.
FIG. 19C illustrates an isometric view of an alternative embodiment of a hub assembly having the buttons of FIGS. 19A and 19B.
FIG. 19D illustrates a medical device engaged with the buttons of 19A and 19B.
FIG. 20 illustrates a cross-sectional view of an alternative embodiment of a hub assembly having the buttons of FIGS. 19A and 19B.
FIG. 21 illustrates an alternative embodiment of a button for use with a valve assembly of a hub assembly.
FIG. 22 illustrates a cross-sectional view of an alternative embodiment of a hub assembly having the buttons of FIG. 21.
FIG. 23 illustrates an alternative embodiment of a valve assembly for use with a hub assembly.
FIG. 24 an alternative embodiment of a hub assembly having the valve assembly of FIG. 23.

### DETAILED DESCRIPTION OF THE DRAWINGS AND THE PRESENTLY PREFERRED EMBODIMENTS

Example embodiments are disclosed herein. It is understood, however, that the disclosed embodiments are merely exemplary and may be embodied in various and alternative forms. The figures are not necessarily to scale; some figures may be configured to show the details of a particular component. Therefore, specific structural and functional details disclosed herein are not to be interpreted as limiting but merely as a representative basis for the claims and/or teaching one skilled in the art to practice the embodiments.

Unless defined otherwise, all technical and scientific terms used herein have the same meaning as commonly understood by one of ordinary skill in the art to which this invention pertains.

The terms "patient," "subject," and "recipient" as used in this application may refer to any animal, particularly humans.

The terms "proximal" and "distal" will be used to describe opposing axial ends of a medical device, as well as the axial ends of various component features. The term "proximal" is used to refer to the end of the medical device (or component thereof) that is closest to the operator during use of the system. The term "distal" is used to refer to the end of the medical device (or component thereof) that is initially inserted into the patient, or that is closest to the patient during use.

The term "biocompatible" refers to a material that is substantially non-toxic in the in vivo environment of its intended use, and that is not substantially rejected by the patient's physiological system (i.e., is non-antigenic). This can be gauged by the ability of a material to pass the biocompatibility tests set forth in International Standards Organization (ISO) Standard No. 10993 and/or the U.S. Pharmacopeia (USP) 23 and/or the U.S. Food and Drug Administration (FDA) blue book memorandum No. G95-1, entitled "Use of International Standard ISO-10993, Biological Evaluation of Medical Devices Part 1: Evaluation and Testing." Typically, these tests measure a material's toxicity, infectivity, pyrogenicity, irritation potential, reactivity, hemolytic activity, carcinogenicity and/or immunogenicity. A biocompatible structure or material, when introduced into a majority of patients, will not cause a significantly adverse, long-lived or escalating biological reaction or response, and is distinguished from a mild, transient inflammation which typically accompanies surgery or implantation of foreign objects into a living organism.

The term "medical device" means any object that is itself or that includes a component that is intentionally inserted into the body of a patient as part of a medical treatment, and that comprises a structure adapted for introduction into a patient. The medical device can be a tool or instrument, such as, without limitation, a catheter, a wire guide, a forceps, or a scissors used to affect a surgical procedure at and/or deliver a second medical device to a treatment site in a patient.

The terms "about" and "substantially" are used herein with respect to measurable values and ranges due to expected variations known to those skilled in the art (e.g., limitations and variability in measurements).

The terms "at least one" and "one or more of" an element are used interchangeably and may have the same meaning. These terms, which refer to the inclusion of a single element or a plurality of the elements, may also be represented by the suffix "(s)" at the end of the element. For example, "at least one metal", "one or more metals", and "metal(s)" may be used interchangeably and are intended to have the same meaning.

Referring to the figures, an embodiment of a medical device 10 useful in performing minimally invasive surgery and controlling suction is provided. In example embodiments, the medical device 10 may be used with a scope, such as an ureteroscope, in which the surgeon inserts the scope into a lumen of the medical device 10. The ureteroscope includes an integral optical system, a working channel, and a controller to maneuver the ureteroscope to the target site. The medical device 10, such as an access sheath 12, may include a hub assembly 20, which allows the user to control drainage and suction during use of the medical device 10.

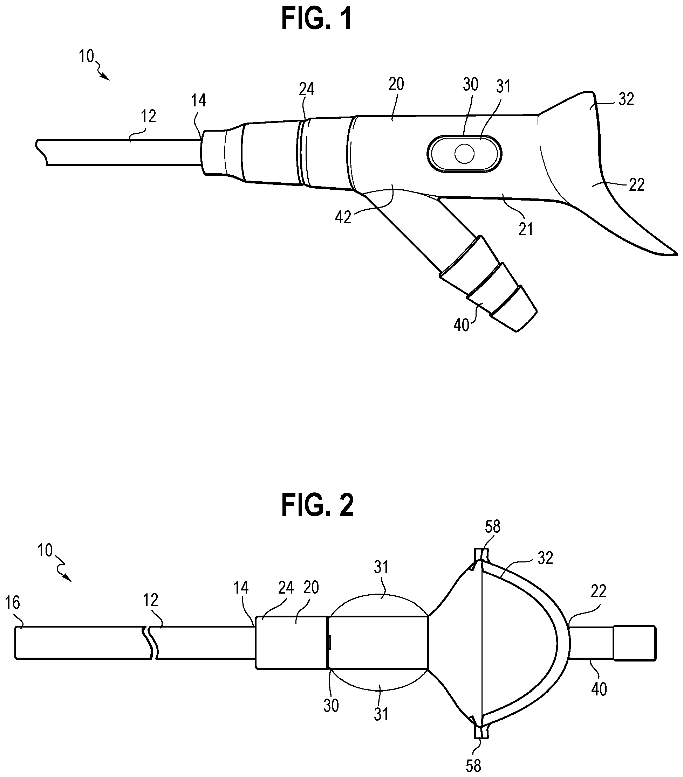

Referring to FIGS. 1 and 2, an example embodiment is illustrated. A medical device 10 comprising an access sheath 12 having a proximal end 14 and a distal end 16 is present. Furthermore, the access sheath 12 includes a lumen (not shown) therethrough. In some embodiments, the proximal end 14 of the access sheath 12 may have a flared configuration. The medical device 10 further includes a hub assembly 20. The hub assembly 20 includes a proximal end 22 and a distal end 24. As shown, the distal end 24 of the hub assembly is connected with the proximal end 14 of the access sheath 12. In some embodiments, the distal end 24 of the hub assembly may be tapered in order to allow for a connection with the access sheath 12. The hub assembly 20 further includes a valve assembly 30, a funnel 32, and a vacuum port 40 connected to a vacuum chamber 42. As shown in FIG. 2, the valve assembly 30 includes valve members disposed on opposite sides of the hub assembly 20. In this embodiment, the valve members are buttons 31. As will be discussed below, the valve members of the valve assembly 30 may be used to open and close the valve during use to control drainage or suction during a procedure. The funnel 32 may be used to introduce additional medical devices for use with the procedure, such as a dilator and/or a ureteroscope. Furthermore, the funnel 32, which is in fluid communication with the lumen of the access sheath 12 will allow for drainage of fluid during the procedure. The vacuum port 40 is in fluid communication with an interior passageway (not shown) of the medical device 10 and a lumen of the access sheath 12. Receiving members 58 are positioned on the periphery of the first opening 55 of the funnel 32. The receiving members 58 comprise a recessed area positioned on opposite sides of the funnel 32. In this embodiment, the receiving members 58 have a generally "U-shaped" configuration, which provides a recess formed between a pair of walls. The receiving members 58 are designed to receive corresponding engaging members to receive the corresponding engaging members of a medical device, such as a dilator clip.

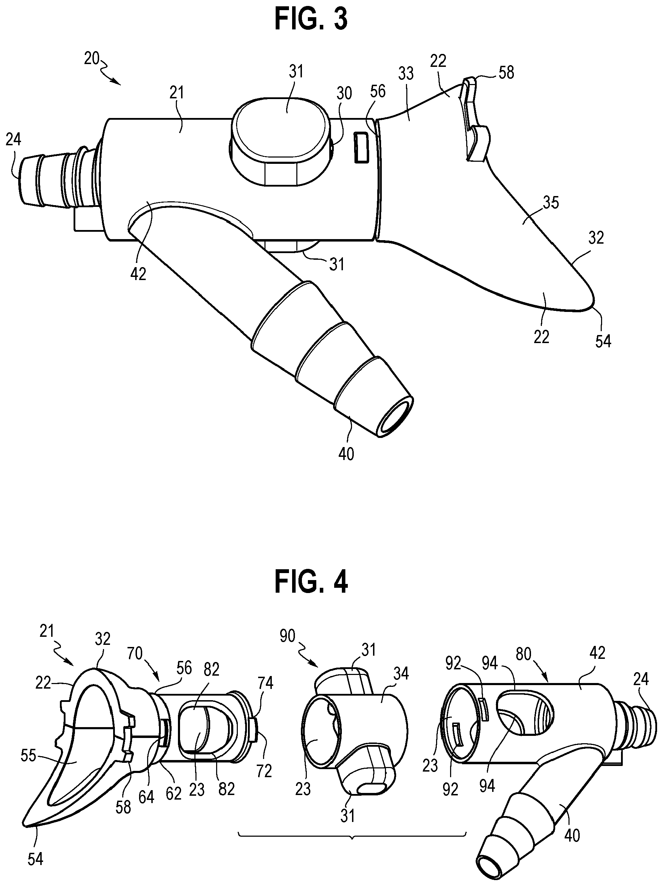

FIG. 3 illustrates an embodiment of the hub assembly 20 for use with the present invention. The hub assembly 20 includes a body 21 that defines an interior chamber or interior passageway, the body 21 of the hub assembly 20 including a proximal end 22 and a distal end 24. In this embodiment, the distal end 24 of the hub assembly 20 is tapered in order to allow for a friction fit connection between the distal end 24 of the hub assembly 20 to the proximal end 14 of the access sheath 12. The friction fit connection allows for embodiments of the access sheath 12 having a proximal end 14 with a flared configuration to be slid and positioned over the distal end 24 of the hub assembly 20. The friction between the proximal end 14 of the access sheath 12 and distal end 24 of the hub assembly 20 in this embodiment allows the access sheath 12 and the hub assembly 20 to remain interconnected. In alternative embodiments, the distal end 24 of the hub assembly 20 may be bonded to the proximal end 14 of the access sheath 12 through the use of an insert and/or overmolding. Additional methods of manufacture, including 3-D printing, may also be considered suitable to form a particular embodiment of the hub assembly 20.

The hub assembly 20 further includes a funnel 32 positioned on the distal end 24 of the hub assembly 20. The funnel 32 includes a proximal end 54 and a distal end 56. As shown, the funnel 32 may include a first portion 33 and a second portion 35 that is elongated relative to the first portion. As will be discussed below, the proximal end 54 includes a first opening 55 that defining a perimeter. The distal end of the funnel 32 may include an attachment member to ensure a connection to the body 21 of the hub assembly. The attachment member may include, but is not limited to, an adhesive, a clamp, a snap-fit connection, a threaded connection, a magnetic connection, a vacuum connection, or combinations thereof. As shown in this embodiment, the attachment member includes a snap-fit connection. Receiving members 58 are positioned on the periphery of the first opening 55 of the funnel 32. The receiving members 58 comprise a recessed area positioned on opposite sides of the funnel 32. In this embodiment, the receiving members 58 have a generally "U-shaped" configuration, which provides a recess formed between a pair of walls. The receiving members 58 are designed to receive corresponding engaging members of another medical device, such as a dilator clip.

The hub assembly 20 further includes a valve assembly 30. In this embodiment, the valve assembly 30 is disposed in an intermediate portion of the body 21 of the hub assembly 20. As shown, the valve assembly 30 includes a valve member which may comprise one or more buttons 31. The buttons 31 provide the user with the ability to open and close the valve assembly 30 in response to needs of the procedure. The buttons 31 allows for unimpeded passage of tools and instruments through the lumen of the hub and sheath when the buttons are in their initial rest position. The valve assembly 30 comprises a pinch valve configuration In other embodiments, the valve assembly 30 may comprise different valve configurations including, but not limited to, disk valves, iris valves, and the like. The hub assembly 20 includes a vacuum port 40 connected to a vacuum chamber 42 within the body 21 of the hub assembly. The vacuum port 40 may include a universal adaptor in order to allow for connection to a vacuum source. The vacuum port 40 is in fluid communication with the interior passageway of the body and the lumen of the access sheath 12.

The hub assembly 20 may comprise any suitable biocompatible material, including, but not limited to, material, including by injection molding and may comprise materials such as an elastomeric polymer or other suitable biocompatible polymer. For example, the hub assembly 20 may be formed from polymers such as polyether-amide block co-polymer (PEBAX^{®}), nylon (polyamides), polyolefins, polyesters, polycarbonates polyurethanes, polydimethyl siloxane, acrylonitrile butadiene styrene (ABS), high density polyethylene (HDPE), rubber, polyisoprene (i.e., synthetic rubbers), and polytetrafluoroethylene. Extrusion or another high temperature processing, such as injection molding, compacting, ultrasonic or radio frequency sinerting, and slot coating can form the hub assembly 20.

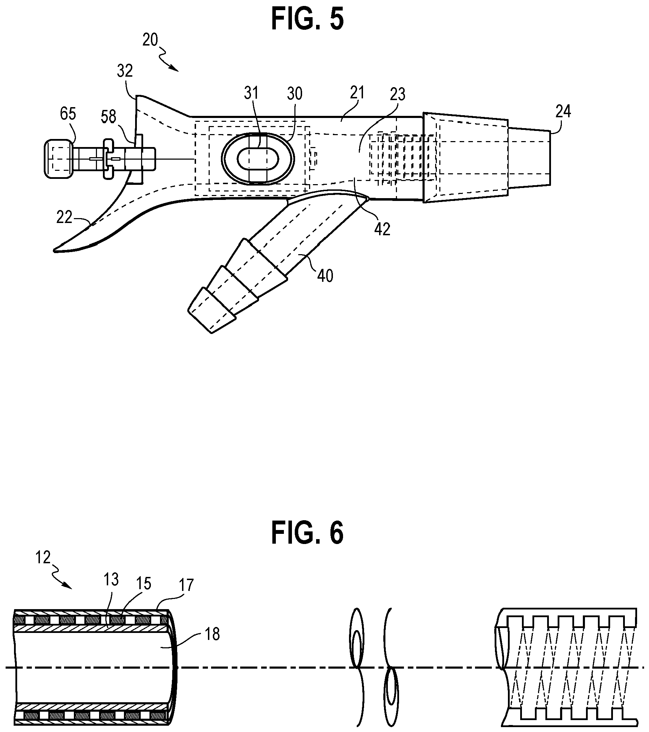

FIG. 4 illustrates an exploded view of the body 21 of the hub assembly 20. As shown in this embodiment, the body 21 defines an interior passageway 23 and includes a proximal end 22 and a distal end 24. The body 21 of the hub assembly 20 comprises three separate sections: a proximal section 70, a distal section 80, and an intermediate section 90. In some embodiments, the hub assembly 20 may comprise more than three sections or less than three sections. In alternative embodiments, the hub assembly 20 may be formed from a single piece of biocompatible material. A funnel 32 is provided on the proximal section 70 of the hub assembly 20. The funnel 32 includes a proximal end 54 and a distal end 56. The proximal end 54 of the funnel 32 may have an elongated configuration and include a first opening 55 defining a first perimeter. The distal end 56 of the funnel 32 includes a second opening (not shown) that has a second perimeter. The second perimeter of the second opening of the funnel 32 has a diameter less than the diameter of the first opening 55. The funnel 32 has a tapered configuration and thus the diameter of the funnel 32 decreases throughout its depth in the distal direction from the first opening 55 to the second opening. The first opening 55 and the second opening of the funnel 32 are in fluid communication with the lumen of the access sheath 12 and the interior passageway of the body 21 of the hub assembly 20. Receiving members 58 are positioned on the periphery of the first opening 55 of the funnel32. The receiving members 58 comprise a recessed area positioned on opposite sides of the funnel 32. The receiving member 58may be used to receive an additional medical device, such as a dilator65, as shown in FIG. 5.

Referring back to FIG. 4, the distal end of the proximal section 70 of the hub assembly 20 has a generally cylindrical shape and a lumen disposed therethrough to help form an interior passageway the body 21 of the hub assembly 20. The proximal section 70 includes a first attachment member 62 having snaps 64 configured to engage with the recesses 92 of the distal section 80 of the hub assembly 20 to ensure connection between the two sections. The distal end of the proximal section 70 includes a second attachment member 72 having snaps 74, to ensure a connection to other components of the body 21 of the hub. As shown, the snaps 74 that are designed to engage with recesses (not shown) positioned on the distal section 80 of the hub assembly 20. The distal end of the proximal section 70 includes a pair of openings 82 positioned on opposite sides. The openings 82 are designed to allow passage of the buttons 31 of the valve assembly into the interior chamber of the body 21 of the hub assembly 20. Accordingly, the diameter of the openings 82 is configured to be larger than the diameter of the buttons 31 of the valve assembly in order to allow the buttons to be positioned or pinched into the interior passageway 23. In some embodiments, the proximal section 70 of the hub assembly 20 may comprise more than two openings or less than two openings to accommodate the number of corresponding buttons 31 of the valve assembly 30.

The distal section 80 of the hub assembly 20 includes the distal end 24 of the hub assembly and a vacuum port 40 connected to a vacuum chamber 42. The vacuum port 40 may include a universal adaptor in order to allow for connection to a vacuum source. Recesses 92 on the distal section 80 of the hub assembly 20 are provided and are designed to engage with the snaps 64 of the first attachment member 62 on the proximal section 70 of the hub assembly 20 to ensure connection between the two sections. The distal section 80 of the body 21 includes a pair of openings 94 positioned on opposite sides. The openings 94 are designed to allow passage of the buttons 31 of the valve assembly into the interior chamber of the body 21 of the hub assembly 20. Accordingly, the diameter of the openings 94 are designed to be larger than the diameter of the buttons 31 of the valve assembly 30 in order to allow the buttons to be positioned orpinched into the interior passageway 23. In some embodiments, the distal section 80 of the hub assembly 20 may comprise more than two openings or less than two openings to accommodate the number of corresponding buttons 31 of the valve assembly 30. Furthermore, the size and diameter of the openings 94 of the distal section 80 of the hub assembly 20 are designed to be the same or substantially similar to the openings 82 on the distal end of the proximal section 70 of the hub assembly.

In this embodiment, the valve assembly 30 is provided on the intermediate section 90 of the body 21 of the hub assembly 20. As shown, the valve assembly 30 includes a valve member that comprises one or more buttons 31 and a sleeve or collar 34. The buttons 31 provide the user with the ability to open and close the valve assembly 30 in response to needs of the procedure. In addition, the sleeve 34 provides a seal around the buttons 31 of the valve assembly 30. As shown in this embodiment, the components of the valve assembly 30 are designed to be placed over the distal end of the proximal section 70. Furthermore, when the proximal section 70 and the distal section 80 are interconnected via the snaps 64 of the first attachment member 62 and the snaps 74 of the second attachment member 74 and the second attachment member, respectively, and their corresponding recesses, the intermediate section 90 is sandwiched between the distal end of the proximal section 70 and the proximal end of the distal section 80 where the buttons 31 of the valve assembly 30 are disposed through the openings 94 of the proximal end of the distal section 80. In some embodiments, the snaps 74 of the second attachment member 72 of the proximal section 70 of the body 21 of the hub assembly 20 may also serve as a support upon which the intermediate section 90 may rest.

The buttons 31 and the sleeve 34 of the valve assembly 30 can be manufactured from any suitable material or combination of materials. The constituent material of the implantable medical device structure may be biodegradable or non-biodegradable. Nonbiodegradable polymers that can be used include, for example, cellulose acetate, cellulose nitrate, silicone, polyethylene terephthalate, polyurethane, polyamide, polyester (e.g. Nylon), polyorthoester, polyanhydride, polyether sulfone, polycarbonate, polypropylene, high molecular weight polyethylene, and polytetrafluoroethylene, or mixtures of these. Biodegradable polymers that can be used include, for example, polylactic acid (PLA), polyglycolic acid (PGA), poly(lactic-co-glycolic acid) (PLGA), polyanhydride, polycaprolactone, polyhydroxybutyrate valerate, or mixtures of these. The buttons 31 and the sleeve 34 of the valve assembly 30 may be manufactured from the same material or each component may comprise different materials. In example embodiments, the buttons 31 and the sleeve 34 are both manufactured from silicone. As shown, the buttons 31 are ergonomically shaped.

In use, the buttons 31 of the valve assembly 30 may be moved laterally (i.e. telescopically) between an open position and a closed position with respect to the longitudinal axis of the hub assembly 20 by the application of force to the buttons 31. In the closed position, the buttons 31 are moved laterally with respect to the longitudinal axis of the hub assembly 20 into the interior passageway 23 of the hub assembly 20 in order to close the pathway to the funnel 32 for drainage. When the valve assembly 30 is in the closed position, the user may be able to apply suction or vacuum through the vacuum port 40. In some embodiments, the buttons 31 may further include inwardly focused features. These features may include a radius of curvature that is substantially similar to the inner surface of the hub assembly 20 in the interior passageway to further seal the interior passageway when the valve assembly 30 is in the closed position. The buttons 31 of the valve assembly 30 are biased to be in the open position, in which the buttons 31 are positioned laterally outward with respect to the longitudinal axis of the hub assembly 20. Accordingly, upon removal of the force applied to the buttons 31, the valve assembly 30 is moved from the closed position to the open position, which allows for the user to proceed with providing any drainage through the interior passageway 23. In addition, the buttons 31 of the valve assembly 30 allows for the distinct process of porting off suction while being ergonomic and designed to minimize fatigue on the user's hand after repeated activation of the suction mechanism.

FIG. 6 illustrates a cross sectional view of an embodiment of an access sheath 12. One class of composite introducer sheaths can occasionally exhibit distal stretch separation breakage when tension in the introducer sheath becomes acute when a practitioner is attempting to withdraw the introducer sheath from a patient after completing a medical procedure. In particular, introducer sheaths made in accordance with co-owned U.S. Pat. No. 5,380,304 include an inner PTFE tube surrounded by a flat wire coil. An outer nylon tube covers that flat wire coil and becomes connected to an outer surface of the PTFE tube between adjacent turns of the flat wire coil. Those skilled in the art will appreciate that the inner PTFE tube helps provide a low friction surface against which medical devices are slid along when being introduced into a patient. The flat wire coil assists in providing reinforcement and inhibits kinking. The outer tube presents a heat formable polyamide material that is advantageously self-leveling for providing a smooth outer surface to the introducer sheath.

From the outside, a medical device 10 according to the present disclosure may look the same as an introducer manufactured according to the teachings of U.S. Pat. No. 5,380,304, and sold for many years under the FLEXOR trademark. However, as shown in FIG. 6, the access sheath 12 includes an inner layer 13, an outer layer 17 disposed about the outer surface of the inner layer 13, an intermediate layer 15 positioned between the inner layer 13, and the outer layer 17 and a lumen 18 disposed therethrough. In this embodiment, the intermediate layer 15 comprises a coil wire reinforcement. In some embodiments, the access sheath 12 may include one or more tie layers to assist with bonding of the inner layer 13, intermediate layer 15, and the outer layer 17. The wall of the inner layer 13 prevents the turns of the coil of the intermediate layer 15 from extending into the lumen 18 of the access sheath 12. The spacing between coil turns along the longitudinal axis of the access sheath 12 may be uniform, and preferably are spaced sufficiently enough that the heat formable nylon connects to a roughened outer surface of the PTFE tube in the spacing between coils. Preferably, the flat wire reinforcement of the intermediate layer 15 is compression fitted around, and applies a compressive force to, the inner layer 13. The inner layer 13 and the outer layer 17 of the access sheath 12 have an inner diameter and an outer diameter, respectively. The outer diameter of the inner layer 13 is less than the outer diameter of the outer layer 17. Specifically, the inner diameter of the inner layer 13 is less than the outer diameter of the outer layer 17 may be about 1.2 Fr or less. In addition, in order to maintain suitable properties for use, the durometer of the outer layer is increased in order to account for the thin walls of the access sheath 12. The coil of the intermediate layer 15 provides adequate surface area coverage through the access sheath 12, which contributes to acceptable buckle-, crush-, and kink resistance during the insertion or removal of the access sheath 12. For example, when the access sheath 12 is introduced over a dilator to through ureter, the layers of the access sheath 12 mitigates kinking during the removal of the dilator or buckling during insertion of the access sheath when a narrowing in the ureter is encountered. The access sheath 12 advantageously has thin walls to decrease the potential for trauma to a patient while maintain a lumen of sufficient size for the passage of tools, instruments, and the evacuation of debris and fluids.

In alternative embodiments, the inner layer 13 and the outer layer 17 may be manufactured using different materials. For example, the outer layer 17 may comprise materials such as, but not limited to, polyethylene, polypropylene, nylon, polyether block amide (PEBA), or polyurethane. The inner layer 17 may be made from a medically suitable material, such as a plastic material with a minimal coefficient of friction, e.g., reinforced plastic, polyimide, fluorinated ethylene propylene (FEP), or polytetrafluoroethylene (PTFE). In some embodiments that comprise one or more tie layers, the tie layer may be made from suitable materials such as HDPE, linear low-density polyethylene (LLDPE), and ethylene vinyl-acetate (EVA). The one or more tie layers may have a lower melting point than the materials provided for the inner layer 13, intermediate layer 15, and outer layer 17.

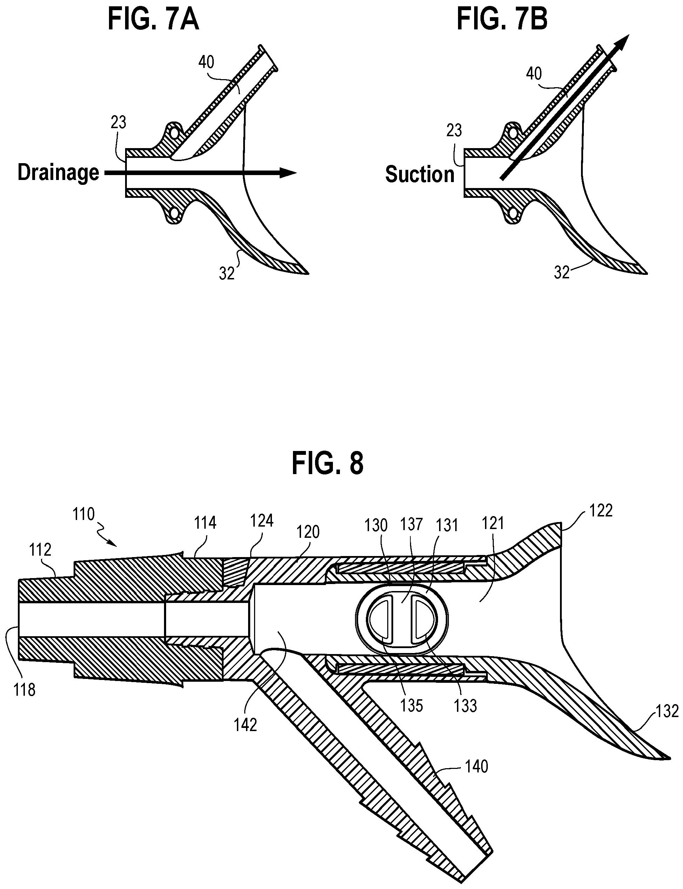

FIGS. 7A and 7B demonstrate the flow of fluid through an embodiment of the hub assembly when the valve assembly is open and closed. FIG. 7A illustrates the flow of fluid when the valve member is in the open position and the valve assembly 30 is open. As shown, a cross section of the hub assembly 20 is shown and illustrating the interior passageway 23 of the body 21 of the hub assembly 20, the funnel 32, and the vacuum port 40. When the valve assembly 30 is in the open position, fluids may be drained through the interior passageway 23 and into the funnel 32 of the hub assembly 20. In addition, while the valve assembly 30 is in the open position, other medical devices may be introduced into the access sheath 12.

FIG. 7B illustrates the flow of fluid when the valve member is in the closed position and the valve assembly 30 is closed. Specifically, when the valve assembly 30 is closed, the user may apply vacuum pressure through the vacuum port 40. Accordingly, the flow of fluid no longer passes into the funnel 32 and instead is diverted in the vacuum port 40. Thus, the user has the ability to utilize forced evacuation of fluid via suction to clear the field-of-view and decrease intrarenal pressure during a procedure such as ureteroscopic lithotripsy.

FIG. 8 illustrates an alternative embodiment of a medical device 110. The medical device 110 comprises an access sheath 112 having a proximal end 114 and a distal end. Furthermore, the access sheath 112 includes a lumen (not shown) therethrough. The proximal end 114 of the access sheath 112 may have a flared configuration. The medical device 110 further includes a hub assembly 120. The hub assembly 120 includes a proximal end 122 and a distal end 124. As shown, the distal end 124 of the hub assembly 120 is connected with the proximal end 114 of the access sheath 112. The distal end 124 of the hub assembly 120 may be tapered in order to allow for a connection with the access sheath 112. The hub assembly 120 further includes a valve assembly 130, a funnel 132, and a vacuum port 140 connected to a vacuum chamber 142. The valve assembly 130 includes valve members disposed on opposite sides of the hub assembly 20 that allow a user to open and close the valve assembly 130 during use to control drainage or suction during a procedure. In the embodiment shown in FIG. 8, the valve members are buttons 131, and the button 131 comprises three components, a proximal portion 133, a distal portion 135, and a strut 137 positioned between the proximal portion 133 and the distal portion 135. In this embodiment, the strut 137 serves to decrease the density of the button 131 as a whole, such that the amount of force needed to compress the proximal portion 133 and the distal portion 135 of the button 131 is also decreased. The funnel 132 may be used to introduce additional medical devices for use with the procedure, such as a ureteroscope. Furthermore, the funnel 132, which is in fluid communication with the lumen of the access sheath 112 will allow for drainage of fluid during the procedure. The vacuum port 140 is in fluid communication with an interior passageway (not shown) of the medical device 10 and a lumen 118 of the access sheath 112.

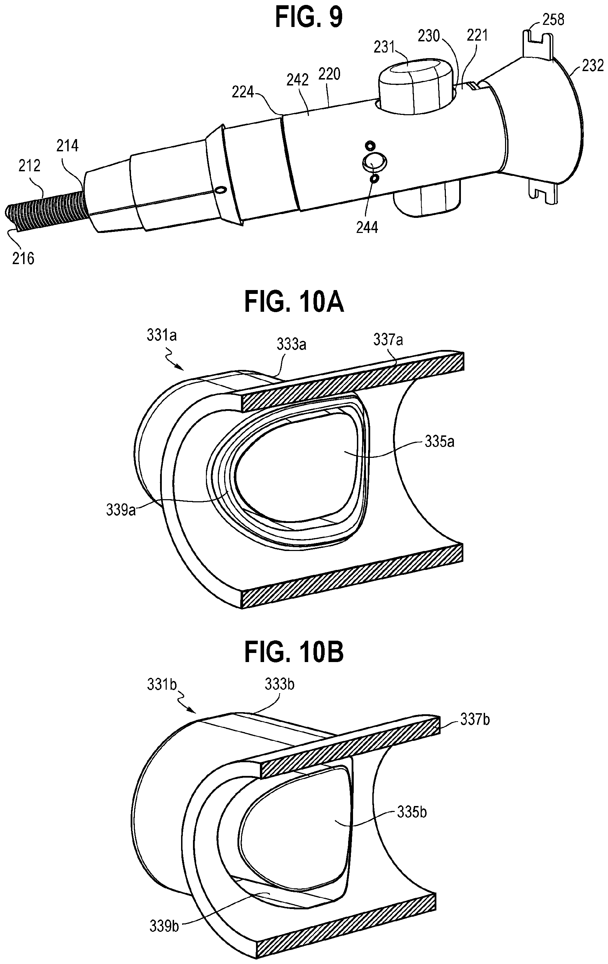

FIG. 9 illustrates an alternative embodiment of a medical device 210. The medical device 210 comprising an access sheath 212 having a proximal end 214 and a distal end 216. Furthermore, the access sheath 212 includes a lumen (not shown) therethrough. The proximal end 214 of the access sheath 212 may have a flared configuration. The medical device 210 further includes a hub assembly 220. The hub assembly 220 includes a proximal end 222 and a distal end 224. As shown, the distal end 224 of the hub assembly 220 is connected with the proximal end 214 of the access sheath 212. The distal end 224 of the hub assembly 220 may be tapered in order to allow for a connection with the access sheath 212. The hub assembly 220 further includes a valve assembly 230, a funnel 232, and a vacuum port (as described in FIGS. 1 and 8) connected to a vacuum chamber 242. The valve assembly 230 includes valve members disposed on opposite sides of the hub assembly 220 that allow a user to open and close the valve assembly 230 during use to control drainage or suction during a procedure. In this embodiment, the valve members are buttons 231. The funnel232 may be used to introduce additional medical devices for use with the procedure, such as a ureteroscope. Furthermore, the funnel 232, which is in fluid communication with the lumen of the access sheath 212 will allow for drainage of fluid during the procedure. Receiving members 258 are positioned on the periphery of the first opening 256 of the funnel 232. The receiving members 258 comprise a recessed area positioned on opposite sides of the funnel 232. In this embodiment, the receiving members 258 have a generally "U-shaped" configuration, which provides a recess formed between a pair of walls. The receiving members 258 are designed to receive corresponding engaging members to receive the corresponding engaging members of the dilator clip.

In the embodiment shown in FIG. 9, the medical device 210 includes at least one vent 244 disposed through the hub assembly 220. The vent 244 allows for pressure to be reduced within the vacuum chamber 242 of the hub assembly 220 when vacuum is being applied. The amount of pressure reduction within the hub assembly 220 may be varied based on the number of vents 244, as well as, the size of the vents. The vent 244 is intended to remain open and is positioned such that it is not blocked by the clinician during use of the access sheath 212. As shown, the vent 244 is positioned on the body of the hub assembly 220 directly opposite of the vacuum port. In alternative embodiments, the vent 244 may be positioned adjacent to the vacuum port, or on the distal most portion of the hub assembly 220 adjacent to the access sheath212. When the vacuum is in operation, air flows through the vent 244, which reduces vacuum pressure in the hub assembly 220. Pressure and flow rate have a direct relationship such that an increase in vacuum pressure leads to a great flow rate of fluid subject to the vacuum pressure. The vent 244 allows for a user to maintain a lower flow rate due to the reduction in vacuum pressure, which provides greater control to the user and mitigates risk to the patient.

FIGS. 10A and 10B illustrates a cross-sectional view of embodiments of the button 331a and 331b. As shown in FIG. 10A, the button 331a has a semi-oblong configuration with a first surface 333a and a second surface 335a. Disposed between the first surface 333 and the second surface 335a is a collar 337a. The collar 337a facilitates attachment of the button 331a to a hub assembly. The first surface 333ahas an ergonomic design for ease of use and a portion of the first surface 333a is designed to remain external of hub assembly. A rim 339a is positioned about the periphery of the second surface 335a of the button 331a. The rim 339a may be used to help maintain a seal of the interior of a hub assembly when the button 331a has not been moved laterally inward to allow for suction. The design of the button 331a allows for unimpeded passage of tools and instruments through the lumen of the hub and sheath when the buttons are in their initial rest position.

As shown in FIG. 10B, the button 331b has a semi-oblong configuration with a first surface 333b and a second surface 335b. Disposed betweenthe first surface 333 and the second surface 335b is a collar 337b. The collar 337a facilitates attachment of the button 331 to a hub assembly. The first surface 333a has an ergonomic design for ease of use and a portion of the first surface 333a is designed to remain external of hub assembly. A slot 339b is positioned about the periphery of the second surface 335b of the button 331b. The oval slot 339b allows the button, when in use, to involute into the interior surface of a hub assembly. The oval slot 339b may include a draft to assist the button 331b in returning to its initial open position once the button 331b is no longer in use. In this embodiment, the button 331b has increased size for additional ergonomic grip and increased functional surface area when in use. When the button 331b is engaged to activate suction, the oval slot 339 allows the second surface 335b of the button 331b to involute within the interior of the hub assembly, thereby creating a seal with a button on the opposing side of the hub assembly. The design of the button 331b allows for unimpeded passage of tools and instruments through the lumen of the hub and sheath when the buttons are in their initial rest position.

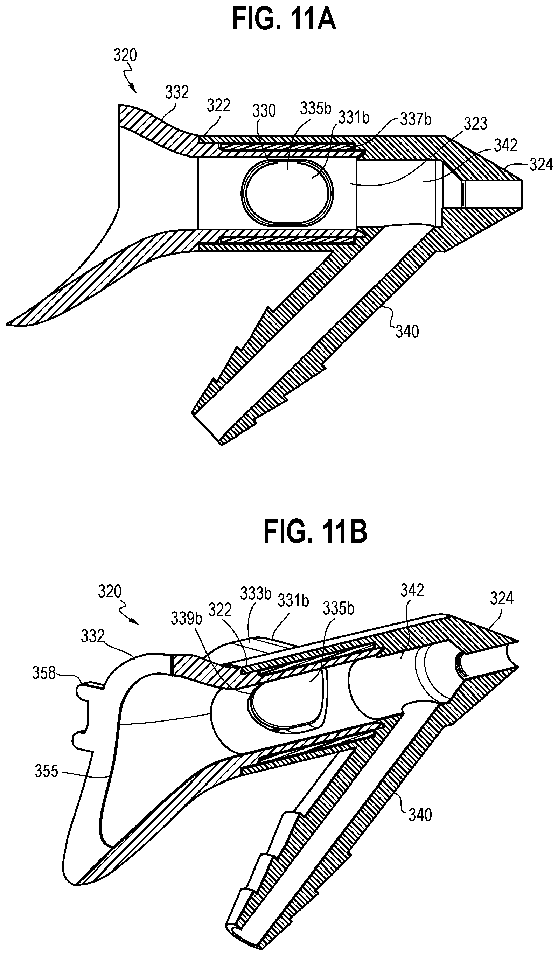

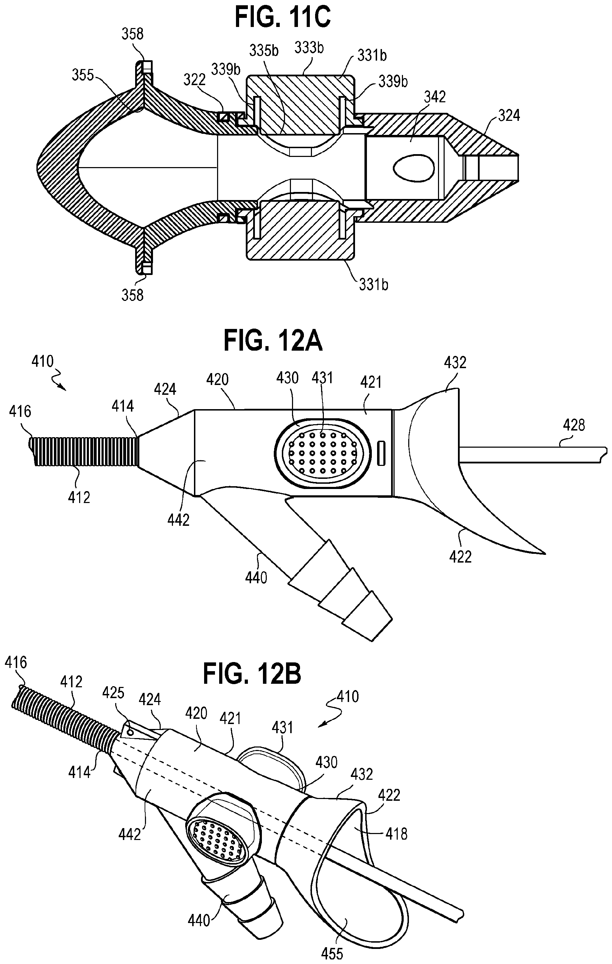

FIGS. 11A-11C illustrate cross-sectional views of an alternative embodiment of a hub assembly 320. The hub assembly 320 includes a proximal end 322 and a distal end 324. The distal end 324 of the hub assembly 320 may be tapered in order to allow for a connection with an access sheath (not shown). The hub assembly 320 further includes a valve assembly 330, a funnel 332, and a vacuum port 340 connected to a vacuum chamber 342. The valve assembly 330 includes valve members, which allows a user to open and close the valve assembly 330 during use to control drainage or suction during a procedure. In this embodiment, the valve members are buttons 331b (as described with respect to FIG. 10B). The button 331b includes a first surface 333b and a second surface 335b. The button 331b is attached to the hub assembly 320 through use of collar 337b. While only one button 331b is shown in the figure, at least one button may be present and positioned directly opposite of the button 331b on the hub assembly 320. The button 331b may be moved laterally (i.e. telescopically) with respect to the longitudinal axis of the hub assembly 320 by the application of force to the buttons into the interior passageway 323 of the hub assembly 320 in order to close the pathway to the funnel332 for drainage. When the valve assembly 330 is in the closed position, the user may be able to apply suction through the vacuum port 340. As shown in FIGS. 11B and 11C, receiving members 358 are positioned on the periphery of the funnel 32. The receiving members 358 comprise a recessed area positioned on opposite sides of a first opening 355 of the funnel 332. In this embodiment, the receiving members 358 have a generally "U-shaped" configuration, which provides a recess formed between a pair of walls. Furthermore, a slot 339b is disposed about the periphery of the second surface 335b to assist with telescopic or radial movement when the button 331b is in use.

FIGS. 12A and 12B illustrates an alternative embodiment of amedical device 410. The medical device 410 comprises an access sheath 412 having a proximal end 414 and a distal end 416. Furthermore, the access sheath 412 includes a lumen 418 therethrough. The proximal end 414 of the access sheath 412 may have a flared configuration. The medical device 410 further includes a hub assembly 420. The hub assembly 420 includes a proximal end 422 and a distal end 424. As shown, the distal end 424 of the hub assembly 420 is connected with the proximal end 414 of the access sheath 412. The distal end 424 of the hub assembly 420 may be chamfered in order to allow for a connection with the access sheath 412. The distal end 424 of the hub assembly 420 further includes one or more suture holes 425 to for securing the hub assembly 420 to a patient. A secondary medical device 428, such as a dilator or a ureteroscope, is disposed through the hub assembly 420. The hub assembly 420 further includes a valve assembly 430, a funnel 432, and a vacuum port 440 connected to a vacuum chamber 442. The valve assembly 430 includes valve members disposed on opposite sides of the hub assembly 420 that allow a user to open and close the valve assembly 430 during use to control drainage or suction during a procedure. In this embodiment, the valve members include buttons 431. The configuration of portions of the buttons 431 disposed within the hub allows for unimpeded passage of tools and instruments through the lumen of the hub and sheath when the buttons are in their initial rest position. The funnel 432 may be used to introduce additional medical devices for use with the procedure, such as a ureteroscope. Furthermore, the funnel 432, which is in fluid communication with the lumen of the access sheath 412 will allow for drainage of fluid during the procedure. In alternative embodiments, receiving members may be positioned on the periphery of the first opening 455 of the funnel 432.

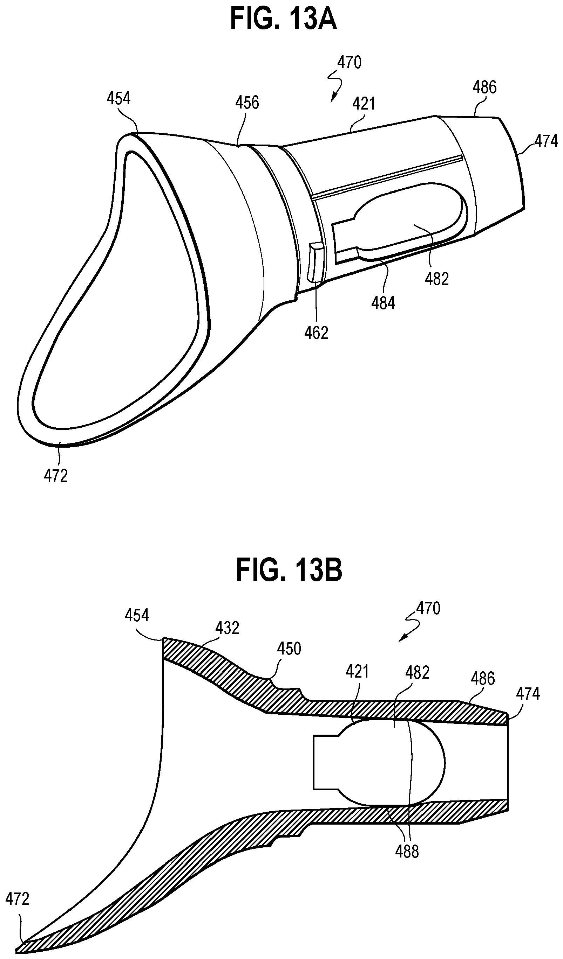

FIG. 13A and 13B illustrate an alternative embodiment of a proximal section 470 of a body 421 of a hub assembly 420. FIG. 13A shows a perspective view of the proximal section 470 having a proximal end 472 and a distal end 474. A funnel 432 is provided at the proximal end 472 of the proximal section 470 of the hub assembly 420. The funnel 432 includes a proximal end 454 and a distal end 456. The proximal end 454 of the funnel 432 includes having a first opening 455 defining a first perimeter. The distal end 456 of the funnel 432 includes a second opening (not shown) that has a second perimeter. The funnel 432 has a tapered configuration and thus the diameter of the funnel 432 decreases throughout its depth in the distal direction from the first opening 455 to the second opening. Snaps 462 may be provided distal of the funnel to facilitate attachment of the proximal section 470 of the hub assembly 420 to a distal section of the of the hub assembly 420. The distal end of the proximal section 470 of the body 421 has a generally cylindrical shape and a lumen disposed therethrough to help form an interior passageway. The distal end 474 of the proximal section 470 includes an opening 482. While one opening 482 is shown, at least one additional opening may be positioned on the opposite side of the distal end of the proximal section 470. A first chamfer 484 is positioned about the first perimeter of opening 482. The first chamfer 484 is provided to help aid and guide valve member of a valve assembly (not shown). The first chamfer 484 also assists in preventing misalignment of the buttons. A second chamfer 486 is present on the distal end of the proximal section 470 of the body 421. The second chamfer 486 aids in assembly and aids in aligning the buttons in the correct position within the proximal section 470 of the body 421. The interior of the proximal section is shown in FIG. 13B. As shown, a closed region 488 is shown surrounding the opening 482. This closed region 488 reduces the area around the opening 482 and within the interior of the hub assembly, which minimizes excessive venting. In addition, the air is blocked from entering the system when the button is compressed, which may increase suction force and reduce user buttonpinch force.

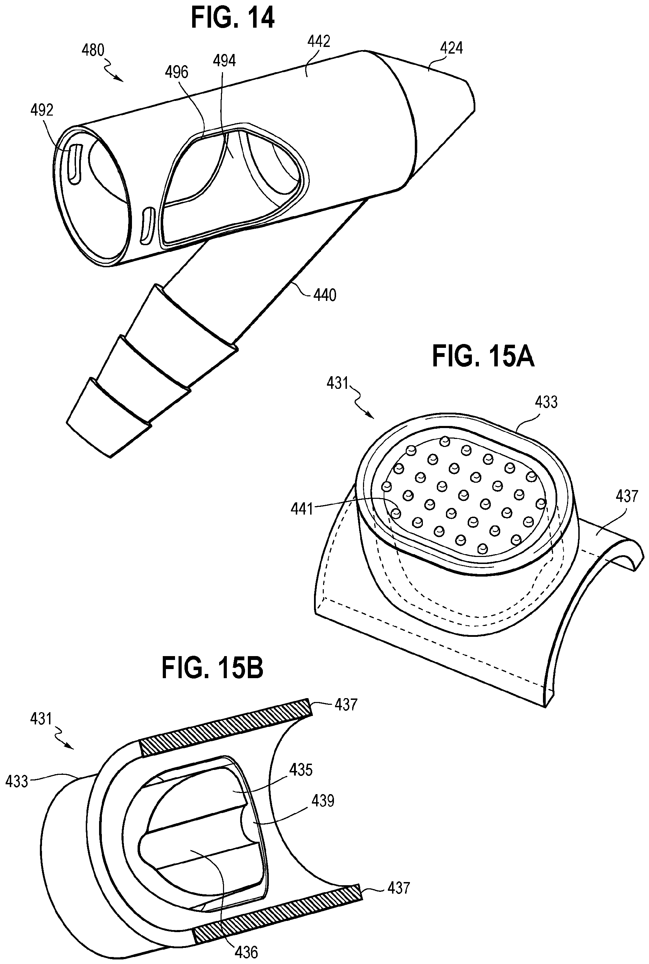

FIG. 14 illustrates an alternative embodiment of a distal section 480 of the body 421 of the hub assembly 420. The distal section 480 of the hub assembly 420 includes the tapered distal end 424 of the hub assembly 420 and a vacuum port 440 connected to a vacuum chamber 442. The vacuum port 440 may include a universal adaptor in order to allow for connection to a vacuum source. Recesses 492 on the distal section 480 of the hub assembly 420 are designed to engage with the snaps 462 of the attachment means positioned on the proximal section 470 of the hub assembly 420 to ensure connection between the two sections. The distal section 480 of the hub assembly 420 includes a pair of openings 494 positioned on opposite sides. The openings 494 are designed to allow passage of the buttons of the valve assembly into the interior chamber of the body 421. A third chamfer 496 is positioned about the openings 494. The third chamfer 496 is provided to help aid and guide buttons of a valve assembly (not shown). The third chamfer 496 allows the buttons to conform to the distal section of the hub assembly 420.

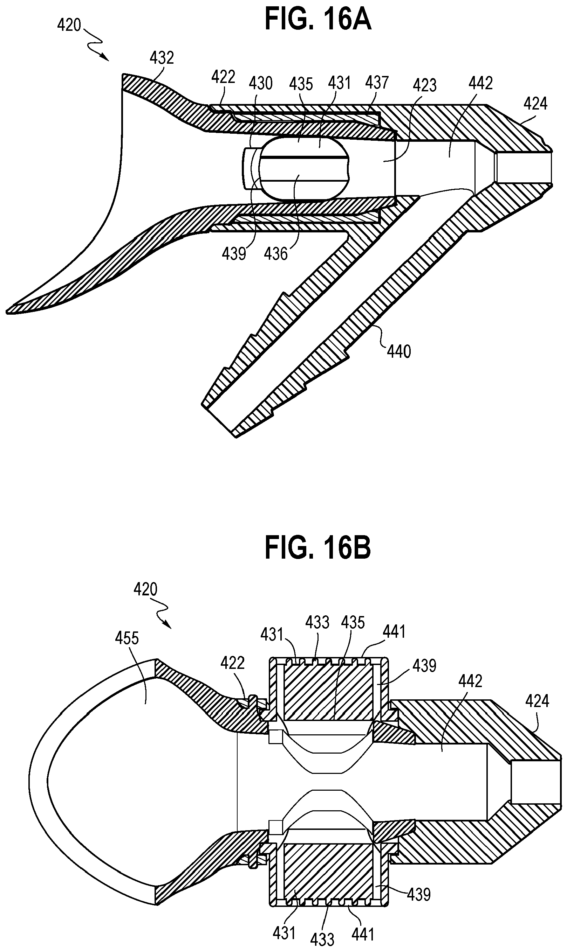

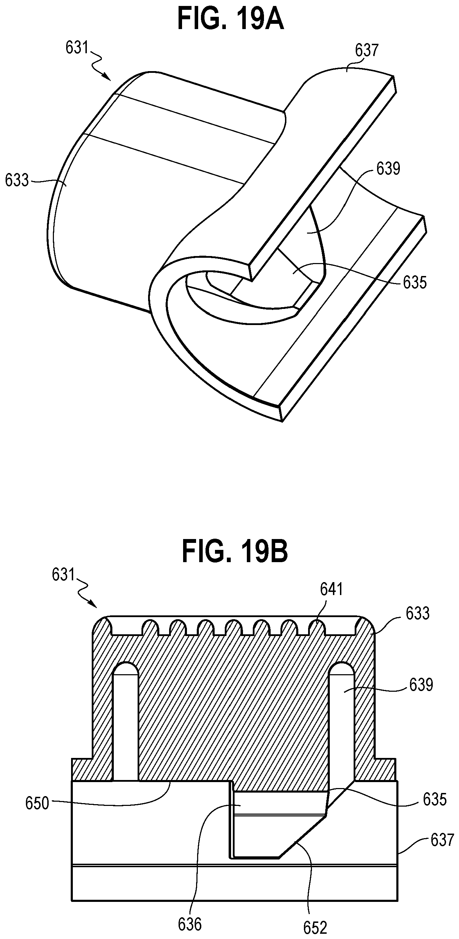

FIG. 15A and 15B illustrate an embodiment of a button 431 for use with the hub assembly 420, which has an external appearance and contours consistent with other views shown in FIGS. 12A, 12B, 16B, and 19B, which includes ornamental design aspects that provides an aesthetic appeal separate from the functional structures and features of the button The button 431 has a semi-oblong configuration with a first surface 433 and a second surface 435. Disposed between the first surface 433 and the second surface 435 is a semi-circular collar 437. The collar 437 is used to attach the button 431 to a hub assembly. In some embodiments, the collar 437 may comprise one part or two parts. In this embodiment, the collar 437 comprises one part. A corresponding collar and button (not shown) is provided on an opposing side. The first surface 433 has an ergonomic design, which may include raised, textured dots 441, for ease of use and a portion of the first surface 433 is designed to remain external of hub assembly, but it should be noted that the functional advantages of grip and other ergonomic features may be realized in other button configurations that look quite different (e.g., with ridges or other textures, with an external geometry other than the obround shape shown, etc.). A slot 439 is positioned about the periphery of the second surface 435 of thebutton431. The slot 439 allows the button, when in use, to involute into the interior surface of a hub assembly. The slot 439 may include a draft to assist the button 431 in returning to its initial rest position once the button 431 is no longer in use. The design of the button 431 allows for unimpeded passage of tools and instruments through the lumen of the hub and sheath when the buttons are in their initial rest position. An indented channel 436 is provided on the second surface of the button 431, as shown in FIG. 15B. With regard to its external appearance, the button is bilaterally symmetrical, including, for example that the perspective view of FIG. 15A will be the same as when viewed from the opposite side. The centered longitudinal cross-section views of FIGS. 16B and 19B show the contours of the upper/outward facing surface including the position and orientation of the raised dots and the surface from which they protrude. As such, all aspects of the buttons 431 visible externally relative to (that is from the outside of) the hub assembly can be appreciated to present an aesthetic appearance.

When the button 431 is engaged to activate suction, the slot 439 allows the second surface 435 of the button 431 to involute within the interior of the hub assembly, thereby creating a seal with a button on the opposing side of the hub assembly. When used without an instrument positioned within the hub assembly 420, the channel 436 acts as a vent to reduce is pressure in the hub assembly 420, while maintaining a sufficient seal for suction. When used with an instrument positioned within the hub assembly, the channel 436 allows for the button 431 to substantially conform around the instrument while also allowing for venting through the channel 436. The venting allows for a user to maintain a lower flow rate due to the reduction in vacuum pressure, which provides greater control to the user and mitigates risk to the patient.

FIGS. 16A and 16B illustrate cross-sectional views of the hub assembly 420. The hub assembly 420 includes a proximal end 422 and a distal end 424. The distal end 424 of the hub assembly 420 may be tapered or chamfered in order to allow for a connection with an access sheath (not shown). The hub assembly 420 further includes a valve assembly 430, a funnel 432, and a vacuum port 440 connected to a vacuum chamber 442. The valve assembly 430 includes buttons 431 (as described with respect to FIGS. 15A and 15B), which allows a user to open and close the valve assembly 430 during use to control drainage or suction during a procedure. The button431 includes a first surface 433 and a second surface 435. The buttons 431 are attached to the hub assembly 420 through use of collar 437. The buttons 431 may be moved laterally (i.e. telescopically) with respect to the longitudinal axis of the hub assembly 420 by the application of force to the buttons into the interior passageway 423 of the hub assembly 420 in order to close the pathway to the funnel 432 for drainage. When the valve assembly 430 is in the closed position, the user may be able to apply suction through the vacuum port 440. As shown, a slot 439 is disposed about the periphery of the second surface 435 to assist with telescopic or radial movement when the button 431 is in use. When used without an instrument positioned within the hub assembly, the channel 436 acts as a vent to reduce is pressure in the hub assembly, while maintaining a sufficient seal for suction.

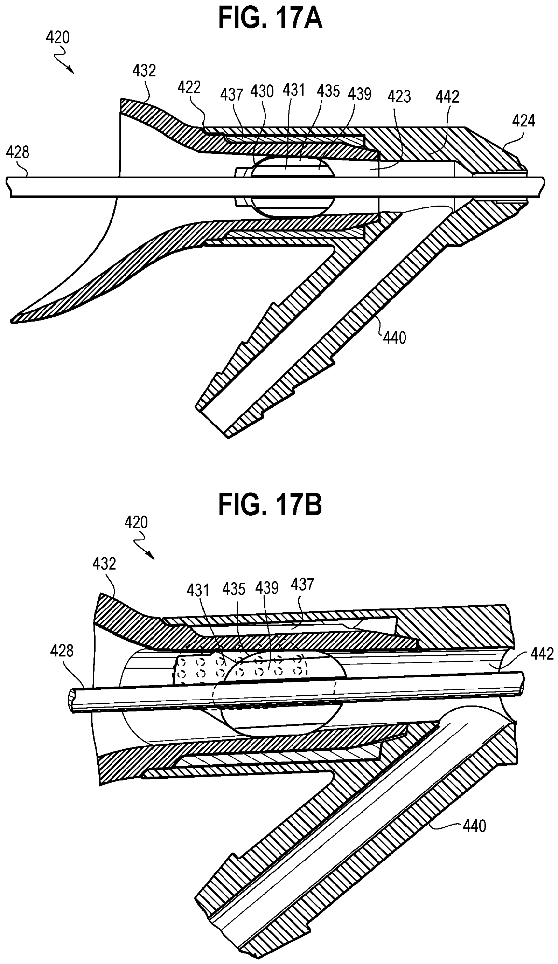

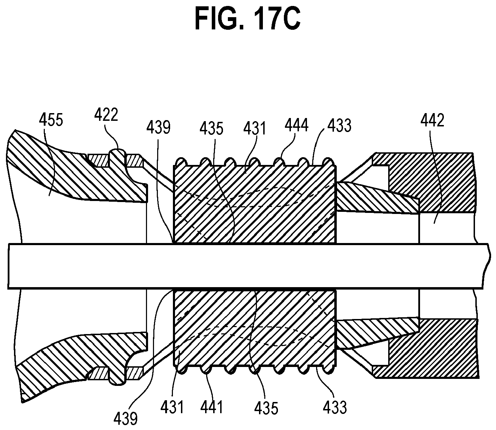

FIGS. 17A-17C illustrate cross-sectional views of the hub assembly 420 when a secondary medical device 428 is disposed within the hub assembly 420. As shown, as the button 431 has been moved laterally inward into the body 421 of the hub assembly 420 to engage the secondary medical device 428. When the button 431 is engaged to activate suction, the oval slot 439 allows the second surface 435 of the button 431 to involute within the interior of the hub assembly 420, thereby creating a seal with a button on the opposing side of the hub assembly. When used with an instrument positioned within the hub assembly, the channel 436 allows for the button 431 to substantially conform around the instrument while also allowing for optional venting through the channel 436. The optional venting allows for a user to maintain a lower flow rate, as desired, due to the reduction in pressure, which provides greater control to the user and mitigates risk to the patient.

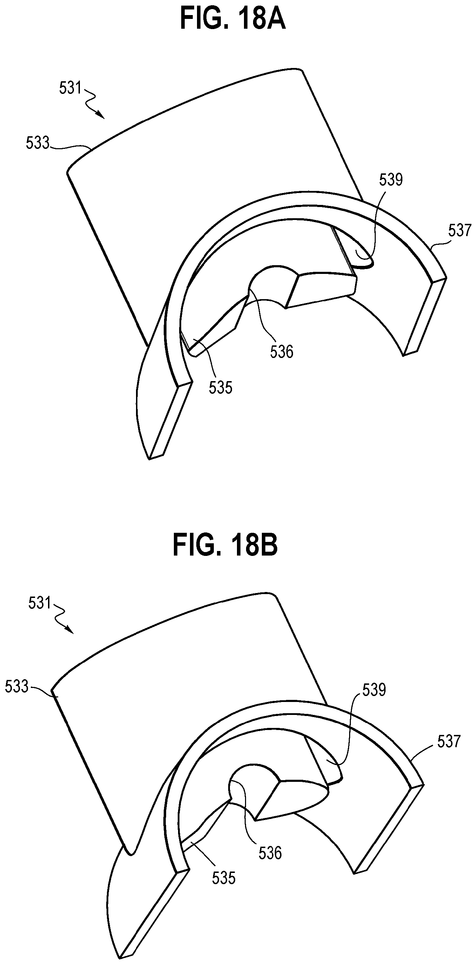

FIGS. 18A and 18B illustrate embodiments of a button 531 for use with a hub assembly 420. The button 531 has a semi-oblong configuration with a first surface 533 and a second surface 535. Disposed between the first surface 533 and the second surface 535 is a semi-circular collar 537. The collar 537 is used to attach the button 531 to a hub assembly 420. In some embodiments, the collar 537 may comprise one part or two parts. In this embodiment, the collar 537 comprises one part. A corresponding collar and button (not shown) is provided on an opposing side. The first surface 533 has an ergonomic design, which may include raised, textured dots, for ease of use and a portion of the first surface 533 is designed to remain external of hub assembly. A slot 539 is positioned about the periphery of the second surface 535 of the button 531. The slot 539 allows the button, when in use, to involute into the interior surface of a hub assembly. The slot 539 may include a draft to assist the button 531 in returning to its initial rest position once the button 531 is no longer in use. The button 531 allows for unimpeded passage of tools and instruments through the lumen of the hub and sheath when the buttons are in their initial rest position. An indented channel 536 is provided on the second surface of the button 531. When the button 531 is used with an instrument positioned within the hub assembly, the channel 536 allows for the button 531 to conform around the instrument while also allowing for optional venting through the channel 536. The optional venting allows for a user to maintain a lower flow rate due to the reduction in pressure, which provides greater control to the user and mitigates risk to the user and patient. The size of the channel 536 may be designed based on the size of the outer diameter of secondary medical devices, such as nephroscopes for use with percutaneous (PCNL) procedures, that are introduced through the hub assembly 420 and an accompanying access sheath. In some embodiments, the size of the channel 536 may range from 8 Fr to 20 Fr.

In addition, the configuration of the channel 536 may be designed based on the configuration of the medical device that will be introduced through the hub assembly 420 and an accompanying access sheath. For example, the embodiments of FIG. 18A and 18B depict buttons having channels 536 of the same size, for example, 9 Fr, but have different configurations. As shown, in the embodiment of FIG. 18A, the position of the channel 536 is at about one-fourth of the diameter of the second surface 535 of the button 531. In the embodiment of FIG. 18B, the position of the channel 536 is at about one-half of the diameter of the second surface 535. Thus, the embodiment of FIG. 18A may be used to accommodate a medical device having a first configuration, while the embodiment of FIG. 18B may be used to accommodate a medical device having a second configuration different from the first configuration. Accordingly, the channel 536 of the button 531 may accommodate medical devices having differing configurations.

FIG. 19A illustrates an embodiment of a button 631 for use with the hub assembly 420. As shown inFIG. 19A, the button 631 has a semi-oblong configuration with a first surface 633 and a second surface 635. In some embodiments, the button 631 may include a semi-circular collar 637. The collar 637 facilitates attachment of the button 631 to a hub assembly. The first surface 633 has an ergonomic design for ease of use and a portion of the first surface 633 is designed to remain external of hub assembly. The second surface 635 is designed to be positioned within the interior of the hub assembly. A slot 639 is positioned about the periphery of the second surface 635 of the button 631.

FIG. 19B illustrates a cross-section the button 631. The first surface 633 includes raised, textured dots 641 (similar to the embodiment shown in FIGS. 15A and 15B), for ease of use. The second surface 635 includes two components: a flattened region 650 and an extended region 652. As shown in the embodiment of FIG. 19B, an indented channel 636 is provided on the extended region 652 of the second surface 635 of the button. When the button 631 is used with an instrument positioned within a hub assembly, the channel 636 allows for the button 631 to conform around the instrument while also allowing for venting through the channel 636. In alternative embodiments, the extended region 652 may have a chamfered configuration. The design of the button 631 allows for unimpeded passage of tools and instruments through the lumen of the hub and sheath when the buttons are in their initial rest position. The design of the button 631 may also be designed to provide venting, which allows for a user to maintain a lower flow rate due to the reduction in pressure, which provides greater control to the user and mitigates risk to the patient.

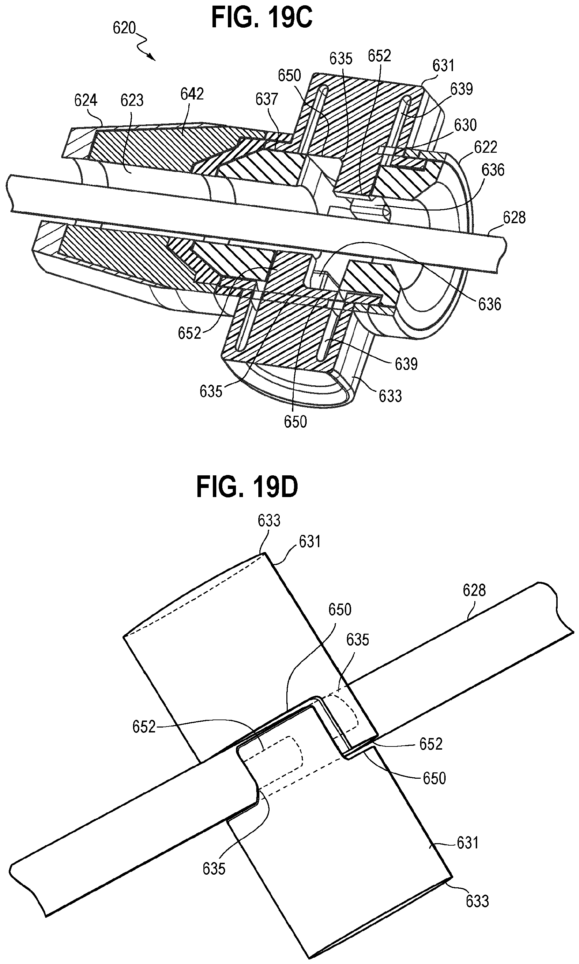

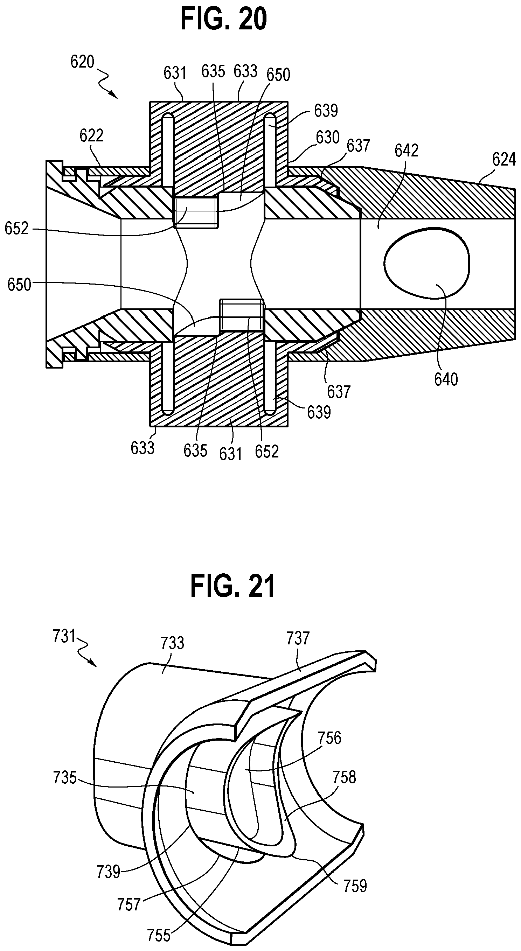

FIG. 19C illustrates an isometric view of an embodiment of a hub assembly 620. The hub assembly 620 includes a proximal end 622 and a distal end 624. The distal end 624 of the hub assembly 620 may be tapered or chamfered in order to allow for a connection with an access sheath (not shown). The hub assembly 620 further includes a valve assembly 630 and a vacuum chamber 642. As with previous embodiments, the hub assembly 620 may include a funnel (not shown in FIG. 20 for the sake of clarity). The valve assembly 630 includes valve members, which allows a user to open and close the valve assembly 630 during use to control drainage or suction during a procedure. In this embodiment, the valve members are buttons 631. The button 631 includes a first surface 633 and a second surface 635. In this embodiment, the second surface 635 includes two components: a flattened region 650 and an extended region 652. As shown, the extended region 652 includes an indented channel 636. The buttons 631 are attached to the hub assembly 620 through use of a collar 637. The buttons 631 may be moved laterally (i.e. telescopically) with respect to the longitudinal axis of the hub assembly 620 by the application of force to the buttons into the interior passageway 623 of the hub assembly 620 in order to close the pathway to the funnel for drainage. A second medical device 628, such as a ureteroscope, is disposed through the hub assembly 620. When the valve assembly 630 is in the closed position, the user may be able to apply suction through a vacuum port (not shown) into the vacuum chamber 642. A slot 639 is disposed about the periphery of the second surface 635 to assist with telescopic or radial movement when the button 631 is in use.

FIG. 19D illustrates a medical device 628 engaged with the buttons 631. Upon application of force in a radial direction towards the medical device 628, the design of the buttons 631 allow for the extended regions 652 of opposing buttons to engage with the respective flattened regions 650 of each of the buttons 631. Thus, the buttons 631 interlock about an outer surface of the medical device 628 and form a seal.

FIG. 20 illustrates a cross-sectional view of an embodiment of a hub assembly 620. The hub assembly 620 includes a proximal end 622 and a distal end 624. The distal end 624 of the hub assembly 620 may be tapered or chamfered in order to allow for a connection with an access sheath (not shown). The hub assembly 620 further includes a valve assembly 630 and a vacuum chamber 642. As with previous embodiments, the hub assembly 620 may include a funnel (not shown in FIG. 20). The valve assembly 630 includes buttons 631 which allows a user to open and close the valve assembly 630 during use to control drainage or suction during a procedure. The button 631 includes a first surface 633 and a second surface 635. In this embodiment, the second surface 635 includes two components: a flattened region 650 and an extended region 652. As shown, the extended region 652 is chamfered. The buttons 631 are attached to the hub assembly 620 through use of a collar 637. The buttons 631 may be moved laterally (i.e. telescopically) with respect to the longitudinal axis of the hub assembly 620 by the application of force to the buttons into the interior passageway 623 of the hub assembly 420 in orderto close the pathway to the funnel 632 for drainage. When the valve assembly 630 is in the closed position, the user may be able to apply suction through a vacuum port 640 into the vacuum chamber 642. A slot 639 is disposed about the periphery of the second surface 635 to assist with telescopic or radial movement when the button 631 is in use.

FIG. 21 illustrates an embodiment of a button 731 for use with the hub assembly 420. The button 731 has a semi-oblong configuration with a first surface 733 and a second surface 735. Disposed between the first surface 733 and the second surface 735 is a collar 737. The collar 737 is used to attach the button 731 to a hub assembly 420. In some embodiments, the collar 737 may comprise one part or two parts. In this embodiment, the collar 737 comprises one part. As shown in this embodiment, the ends of the collar 737 are chamfered. In these embodiments, the chamfered ends of the collar 737 may assist in better vacuum retention within the hub assembly 420. A slot 739 is positioned about the periphery of the second surface 735 of the button 731. The slot 739 allows the button, when in use, to involute into the interior surface of a hub assembly. The slot 739 may include a draft to assist the button 731 in returning to its initial rest position once the button 731 is no longer in use. A corresponding collar and button (not shown) is provided on an opposing side. The design of the button 731 allows for unimpeded passage of tools and instruments through the lumen of the hub and sheath when the buttons are in their initial rest position.

As shown, the first surface 733 has an ergonomic design, which may include raised, textured dots, for ease of use and a portion of the first surface 733 is designed to remain external of hub assembly. The second surface 735 includes an extended region 752 having a generally oval configuration. In this embodiment, the extended region 752 includes walls 755 defining an interior space 756 of the extended region 752. The walls 755 include a first end 757 and a second end 758 having an edge 759. The edge 759 of the second end 758 is chamfered, which contributes to vacuum suction within the hub when the button is in use. In addition, the extended region 752 of the second surface 735 allows for the button 731 to conform around a medical device and form a seal.

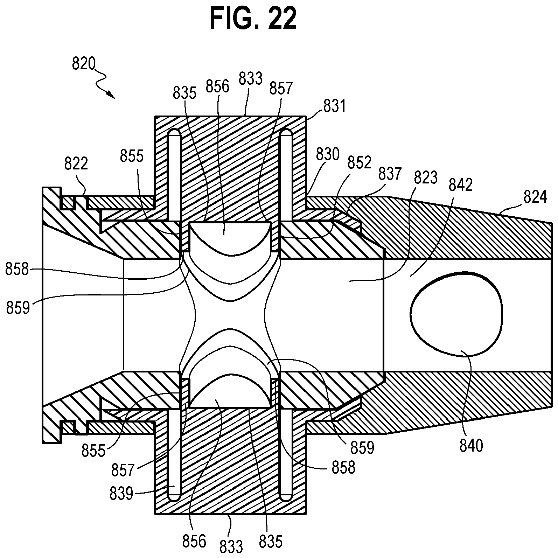

FIG. 22 illustrates a cross-sectional view of an embodiment of a hub assembly 820. The hub assembly 820 includes a proximal end 822 and a distal end 824. The distal end 824 of the hub assembly 820 may be tapered or chamfered in order to allow for a connection with an access sheath (not shown). The hub assembly 820 further includes a valve assembly 830 and a vacuum chamber 842. As with previous embodiments, the hub assembly 820 may include a funnel (not shown in FIG. 22). The valve assembly 830 includes valve members which allows a user to open and close the valve assembly 830 during use to control drainage or suction during a procedure. In this embodiment, the valve members are buttons 831. The button 831 includes a first surface 833 and a second surface 835. In this embodiment, the second surface 835 includes an extended region 852. The extended region 852 includes walls 855 having a first end 857 and a second end 858 having an edge 859. The edge 859 of the second end 858 is chamfered. The buttons 831 are attached to the hub assembly 820 through use of a collar 837. The buttons 831 may be moved laterally (i.e. telescopically) with respect to the longitudinal axis of the hub assembly 820 by the application of force to the buttons into the interior passageway 823 of the hub assembly 820 in orderto close the pathway to the funnel 832 for drainage. When the valve assembly 830 is in the closed position, the user may be able to apply suction through a vacuum port 840 into the vacuum chamber 842. A slot 839 is disposed about the periphery of the second surface 835 to assist with telescopic or radial movement when the button 831 is in use.

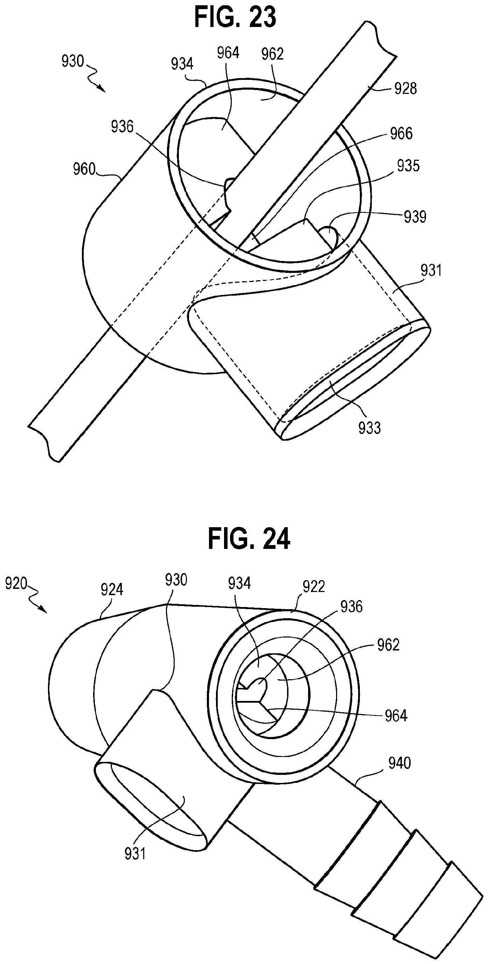

FIG. 23 illustrates an embodiment of a valve assembly 930 for use with a hub assembly. The valve assembly 930 includes a valve member comprising a button 931 and a sleeve 934. In some embodiments, the button 931 and the sleeve 934 may be integrally formed. In other embodiments, the button 931 and the sleeve 934 may be separate components and coupled together. As shown, this embodiment includes a single button 931, which may reduce the amount of force necessary to activate the valve assembly 930 when in use. The button 931 has a semi-oblong configuration with a first surface 933 and a second surface 935. The first surface 933 has an ergonomic design for ease of use and a portion of the first surface 933 is designed to remain external of hub assembly. A slot 939 is positioned about the periphery of the second surface 935 of the button 931. The slot 939 allows the button, when in use, to involute into the interior surface of a hub assembly. The slot 939 may include a draft to assist the button 931 in returning to its initial rest position once the button 931 is no longer in use. An indented channel (as described in FIG. 15A and 15B) may be provided on the second surface 935 of the button.

In the embodiment shown in FIG. 23, the sleeve 934 has an exterior surface 960 and an interior surface 962. In this embodiment, the sleeve 934 has a generally cylindrical configuration and defines an opening 966. A secondary medical device 928 is positioned within the sleeve 934 through the opening 966. The interior surface 962 of the sleeve 934 includes an extended region 964. As shown, the extended region 964 is positioned opposite the button 931. An indented channel 936 is provided on the extended region 964 of the sleeve 934. In some embodiments, the extended region 964 may have a configuration similar to the second surface 935 of the button 931. In alternative embodiments, the extended region 964 may have a configuration different than the second surface 935 of the button 931. In some embodiments, the extended region 964 is configured to remain stationary within the interior surface 962 of the sleeve 934. As shown, this embodiment includes a single button 931, which may reduce the amount of force necessary to activate the valve assembly 930 when in use.

FIG. 24 illustrates an embodiment a hub assembly 920. The hub assembly 920 includes a proximal end 922 and a distal end 924. The hub assembly 920 further includes a valve assembly 930 and vacuum port 940 connected to a vacuum chamber 942. As with previous embodiments, the hub assembly 920 may include a funnel (not shown). The valve assembly 930 includes a button 931 and a sleeve 934 (as described with respect to FIG. 23). The button 931 allows a user to open and close the valve assembly 930 during use to control drainage or suction during a procedure. The button 931 includes a first surface 933 and a second surface (not shown). The sleeve 934 has a generally circular configuration and has an interior surface 962 including an extended region 964. As shown, the extended region 964 is positioned opposite the button 931. An indented channel 936 is provided on the extended region 964 of the sleeve 934. The button 931 may be moved laterally (i.e. telescopically) with respect to the longitudinal axis of the hub assembly 920 by the application of force to the button into an interior passageway of the hub assembly 920 in order to close a pathway for drainage. When the valve assembly 930 is in the closed position, the user may be able to apply suction through the vacuum port 940.

Although the subject matter has been described in language specific to structural features and/or methodological acts, it is to be understood that the subject matter defined in the appended claims is not necessarily limited to the specific features or acts described. Rather, the specific features and acts are disclosed as illustrative forms of implementing the claims.

One skilled in the art will realize that a virtually unlimited number of variations to the above descriptions are possible, and that the examples and the accompanying figures are merely to illustrate one or more examples of implementations.

It will be understood by those skilled in the art that various other modifications can be made, and equivalents can be substituted, without departing from claimed subject matter. Additionally, many modifications can be made to adapt a particular situation to the teachings of claimed subject matter without departing from the central concept described herein. Therefore, it is intended that claimed subject matter not be limited to the particular embodiments disclosed, but that such claimed subject matter can also include all embodiments falling within the scope of the appended claims, and equivalents thereof.

In the detailed description above, numerous specific details are set forth to provide a thorough understanding of claimed subject matter. However, it will be understood by those skilled in the art that claimed subject matter can be practiced without these specific details. In other instances, methods, devices, or systems that would be known by one of ordinary skill have not been described in detail so as not to obscure claimed subject matter.

Reference throughout this specification to "one embodiment" or "an embodiment" can mean that a particular feature, structure, or characteristic described in connection with a particular embodiment can be included in at least one embodiment of claimed subject matter. Thus, appearances of the phrase "in one embodiment" or "an embodiment" in various places throughout this specification are not necessarily intended to refer to the same embodiment or to any one particular embodiment described. Furthermore, it is to be understood that particular features, structures, or characteristics described can be combined in various ways in one or more embodiments. In general, of course, these and other issues can vary with the particular context of usage. Therefore, the particular context of the description orthe usage of these terms can provide helpful guidance regarding inferences to be drawn for that context.

## Claims

1. An introducer assembly, comprising:
a sheath comprising a proximal end, a distal end, and at least one lumen disposed therethrough; and
a hub assembly interconnected with the proximal end of the sheath, the hub assembly comprising:
a body defining an interior passageway in fluid communication with the lumen of the sheath, the body having a proximal end and a distal end;
a valve assembly positioned within the interior passageway of the body, the valve assembly comprising a valve member, the valve member being at least partially movable from a first position to a second position; and
a vacuum chamber positioned distal to the valve assembly and in fluid communication with the interior passageway of the body and the lumen of the sheath, the vacuum chamber including a vacuum port.

2. The introducer assembly of claim 1, wherein the valve member of the valve assembly is flexible.

3. The introducer assembly of claim 1 or 2, wherein the valve member comprises a first button and a second button for example wherein the valve member comprises a first button comprising a proximal portion, a distal portion and a first strut positioned between the proximal portion and the distal portion and the second button comprises a proximal portion a distal portion and a first strut positioned between the proximal portion and the distal portion.

4. The introducer assembly of any preceding claim, wherein the valve member is laterally moveable with respect to a longitudinal axis of the hub assembly.

5. The introducer assembly of any preceding claim, wherein the valve member has a radius of curvature that engages an interior surface of interior chamber of the body.

6. The introducer assembly of any preceding claim, wherein the valve assembly comprises silicone.

7. The introducer assembly of any preceding claim, wherein the sheath comprises two or more layers, for example wherein the sheath comprises an inner layer having an inner diameter and an outer layer having an outer diameter, the outer diameter of the outer layer being greater than the inner diameter of the inner layer by at most 1 Fr.

8. The introducer assembly of claim 7 wherein at least one coil is embedded between the inner layer and the outer layer.

9. A hub assembly for an introducer assembly, comprising:
a body defining an interior passageway, the body having a proximal end and a distal end;
a valve assembly positioned within the interior passageway of the body, the valve assembly comprising,
a valve collar positioned within the interior passageway of the body and defining an interior passageway; and
a valve member connected to the valve collar, the valve member being at least partially movable from a first position to a second position with respect to the valve collar; and
a vacuum chamber interconnected with the distal end of the valve collar and in fluid communication with the interior chamber of the body, the vacuum chamber including a vacuum port.

10. The hub assembly of claim 9, wherein the valve member of the valve assembly is flexible.

11. The hub assembly of claim 9 or 10, wherein the valve member comprises a first button having a first surface and a second surface and a second button having a first surface and a second surface, for example wherein the second surface of the first button and the second button includes one of: (a) a channel (b) a flattened region and an extended region; and (c) an extended region having a chamfered edge.

12. The hub assembly of claim 9 or 10 wherein the valve member comprises a first button comprising a proximal portion, a distal portion, and a strut positioned between the proximal portion and the distal portion, and a second button comprising a proximal portion, a distal portion and a strut positioned between the proximal portion and the distal portion.

13. The hub assembly of any of claims 8 to 12, wherein the valve member has a radius of curvature that engages an interior surface of interior chamber of the body.

14. The hub assembly of any of claims 8 to 13, wherein the valve assembly comprises silicone.

15. An introducer assembly, comprising:
a multi-layer sheath comprising a proximal end, a distal end, and at least one lumen disposed therethrough, the multi-layer sheath having an inner layer having an outer diameter, an outer layer having an outer diameter, and at least one coil embedded between the inner layer and the outer layer; and
a hub assembly interconnected with the proximal end of the multi-layer sheath and having a proximal end, a distal end, and a body defining an interior chamber in fluid communication with the lumen of the multi-layer sheath, the body having a proximal end and a distal end;
a valve assembly positioned within the interior chamber of the body, the valve assembly comprising,
a valve collar positioned within the interior chamber of the body and defining an interior passageway, the interior passageway being in fluid communication with the at least one lumen of the sheath;
a valve member connected to the valve collar, the valve member being at least partially movable from a first position to a second position with respect to the valve collar; and
a vacuum chamber positioned distal to the valve assembly and in fluid communication with the interior chamber of the body and the lumen of the sheath, the vacuum chamber including a vacuum port.
